# EUROPEAN PATENT APPLICATION

(11) **EP 2 017 273 A1**
(43) Date of publication of application: **21.01.2009**
(21) Application number: 07380214.2
(22) Date of filing: 18.07.2007
(51) Int. Cl.: C07D 317/20, C07C 229/14, C07D 491/056, C07D 207/273, C07C 229/08, C07D 207/27, C07D 207/267, C07C 227/16, C07C 227/18

(54) **Process for the enantioselective preparation of pregabalin**

(71) Applicant: Laboratorios del Dr. Esteve S.A., 08041 Barcelona (ES)
(72) Inventor: Ortuno, Rosa M., 08031 Barcelona (ES); Izquierdo Sandra, 08206 Sabadell, Barcelona (ES)
(74) Representative: Carpintero Lopez, Francisco

(57) **Abstract**

The invention provides a new enantioselective process for the preparation of (S)-pregabalin, or a pharmaceutical acceptable addition acid salt comprising: acid hydrolysis of the dioxolan ring of a chiral compound of general formula (I) to obtain compound of general formula (II); oxidation of compound (II) to obtain a compound of general formula (III) and transforming compound (III) into compound of general formula (IV); subjecting compound (IV) to basic hydrolysis to obtain a compound of general formula (V) which is reduced to obtain enantiomerically pure S-pregabalin. The invention also provides new chiral intermediates involved in the process.

## Description

### FIELD OF INVENTION

The present invention relates to a process for the enantioselective preparation of (S)-pregabalin, or a salt thereof, in particular a pharmaceutically acceptable salt. The chirality is introduced at the beginning of the process using D-mannitol bisacetonide to obtain the starting compound (S)-4-((*S*)-2,2-dimethyl-1,3-dioxolan-4-yl)pyrrolidin-2-one, optionally protected by an amine protective group, and is maintained through the process. The invention also relates to new chiral intermediates involved in the process.

### BACKGROUND OF THE INVENTION

(*S*)-pregabalin, also known as (*S*)-3-(aminomethyl)-5-methylhexanoic acid, has been developed as a follow-up compound to Gabapentin, NEURONTIN^{®} for use in the treatment of epilepsy, pain, anxiety and social phobia. Both (*S*)- pregabalin, and gabapentin are analogs of 4-aminobutyric acid (GABA), a neurotransmitter that is thought to play a major inhibitory role in the central nervous system (CNS). (*S*)-pregabalin has been approved in US for the treatment of nerve pain associated diabetes and shingles, as of Dec. 31, 2004.

(*S*)-pregabalin has been found to activate GAD (L-glutamic acid decarboxylase), has a dose dependent protective effect on-seizure, and in a CNS-active compound. (*S*)- pregabalin has been found to be useful in anticonvulsant therapy, due to its activation of GAD, promoting the production of GABA, one of the brain's major inhibitory neurotransmitters, which is released at 30 percent of the brains synapses. (*S*)- pregabalin has an analgesic, anti-convulsant and anxiolitic activity.

The pharmacological activity of pregabalin is primarily attributable to the S-enantiomer and thus, several methods have been developed to prepare the S enantiomer of pregabalin substantially free of the R-enantiomer.

Typically, a racemic mixture is synthesized and then subsequently resolved into its R-and S-enantiomers. Different approaches are known in the state of the art like the "malonate" synthesis (see for instance US 5,840,956) or the "Hofmann rearrangement" synthesis (see for instance US 5,616,793), in which the classical method of resolving a racemate is used to obtain (S)-pregabalin. This involves the preparation of a salt with a chiral agent to separate and purify the desired (S)-pregabalin. This involves significant processing, and additional costs associated with the resolving agent. Partial recycle of the resolving agent is feasible but requires additional processing and costs. Moreover the undesired (R)-pregabalin can not be efficiently recycled and is discarded as waste. The maximum yield of pregabalin is thus 50%, since only half of the racemate is of interest. This reduces the effective throughput of the process which is a component of the production cost and capacity.

(S)-pregabalin has alternatively been synthesized by different routes comprising chiral auxiliaries or chiral catalyst.

One approach to the preparation of pregabalin comprises the use of chiral aluminium salen catalyst in the conjugate addition of TMSCN (trimethylsilyl cyanide) to the corresponding precursor α,β-unsaturated imide (Highly enantioselective, catalytic conjugate addition of cyanide to α,β-unsaturated imides" Jacobsen et al., J. Am. Chem. Soc. 2003, 125, 4442-43). While this route affords high enantiomeric purity, it shows practical limitations for large-scale synthesis because they employ costly reagents like the chiral aluminium salen catalyst, and the relative expensive cyanide source TMSCN.

Another process to afford pregabalin ("Diastereoselective Conjugate Addition of Cyanide to α,β-unsaturated oxazolidinones: Enantioslective Synthesis of ent-Pregabalin and Baclofen"; Armstrong A. et al., Synlett, 2006 no. 10, pp 1589-1591) comprises the hydrocyanation of a chiral compound of the following formula (3d): 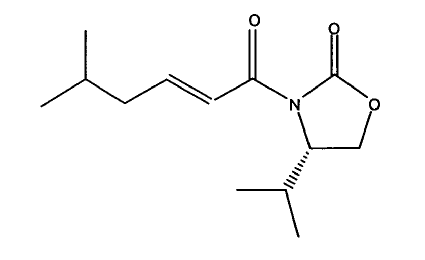 in the presence of samarium (III) isopropoxide as a catalyst which affords the major diastereomer of formula (4d): 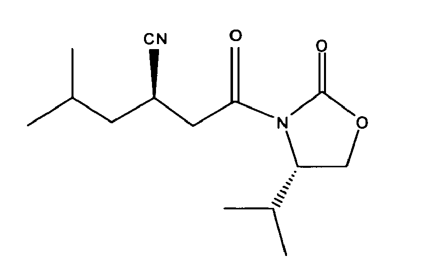 which is thereafter submitted to hydrogenation of the nitrile over platinum oxide with concomitant auxiliary cleavage to yield a lactam, and to the acidic hydrolytic opening of the lactam which yields the ent-pregabalin with retention of the enatiomeric purity.

Although this process directly affords high enantiomeric pure (S)-pregabalin it suffers from some drawbacks, due to the relative high cost of the catalyst system and the synthesis of the chiral costly starting material.

Since (S)-pregabalin is being developed as a commercial pharmaceutical product, there is the need in the state of the art of an alternative, efficient, and cost effective process for its large-scale synthesis, which overcomes at least some of the disadvantages above mentioned.

### SUMMARY OF THE INVENTION

One object of the present invention is thus the provision of a process for the enantioselective preparation of (S)-pregabalin, or a pharmaceutically acceptable acid addition salt.

The inventors have surprisingly discovered that it is possible to prepare (S)-pregabalin from a starting chiral compound of the following formula (I), wherein R is H, or a protective group:

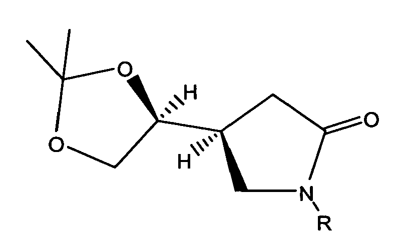

The process also involves new chiral intermediates, which are another object of the present invention. The starting material of formula (I) is advantageously prepared from commercially available D-mannitol bisacetonide and the optical purity is maintained through the process.

Another object of the invention relates to the use of the intermediate of formula (I) in the preparation of (S)-pregabalin or a pharmaceutically acceptable acid addition salt.

A further object relates to a process for preparing (S)-pregabalin, or a pharmaceutically acceptable acid addition salt, from compound of formula (I) which is obtained from D-mannitol bisacetonide.

### DETAILED DESCRIPTION OF THE INVENTION

The present invention relates to a process for enantioselectively preparing pure (S)-pregabalin or a pharmaceutically acceptable acid addition salt.

The process is represented in **Schema 1,** and will be also referred to, hereinafter, as the process of the invention.

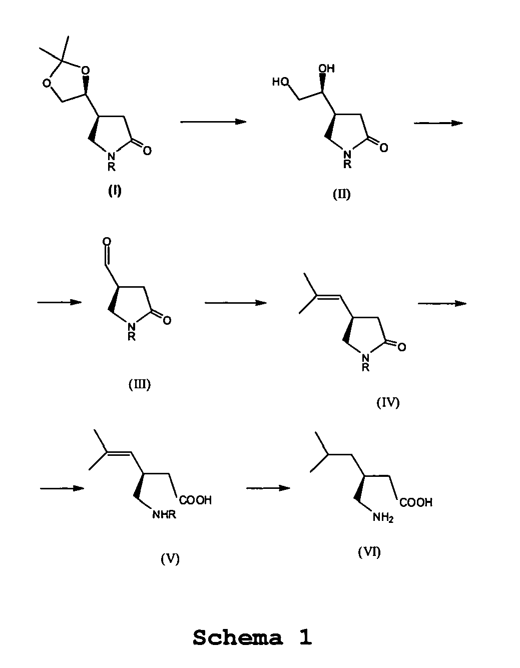

Thus, the process of the invention comprises the reduction of the double bond of a compound of general formula (V), wherein R represents H or a protective group of the amine. Said protective group of the amine, may be any conventional protective group, such as benzyl, Boc, Cbz, 4-methoxybenzyl (PMB), or benzyloxymethyl (BOM).

The reduction is carried out according to one of the following alternatives:
(i) with hydrogen in the presence of a palladium or platinum catalyst,
(ii) by hydrogen transfer from HCO₂NH₄ or cyclohexadiene in the presence of a palladium or platinum catalyst, or
(iii) by hydrogenation in the presence of a palladium or platinum catalyst and HCl.

According to the alternatives (i)-(iii) the palladium or platinum catalyst may be selected from the group consisting of Pd/C, Pd(OH)₂/C, PdCl₂/C, PtO₂/C and their mixtures. Suitable solvents are methanol, ethanol, acetic acid or their mixtures.

In a particular embodiment Pd(OH)₂/C is used as a catalyst in MeOH as solvent and in the presence of an acid, such as HCl to obtain the salt hydrochloride of (S)-pregabalin.

The process of the present invention provides (S)-pregabalin (VI) in its neutral form, or as a pharmaceutically acceptable acid addition salt. Thus the free amine may be converted to the salt for ease handling and administration. Acids commonly employed to form such salts are inorganic acids such as, hydrochloric acid, hydrobromic acid, hydroiodic acid, sulphuric acid, phosphoric acid, and the like, and organic acids, such as p-toluenesulfonic acid, methanesulfonic acid, oxalic acid, p-bromophenylsulfonic acid, carbonic acid, succinic acid, citric acid, benzoic acid, acetic acid and the like. A preferred pharmaceutically acceptable salt is the one formed with hydrochloride acid.
The acid addition salt of (S)-pregabalin may be prepared by adding the corresponding acid to the (S)-pregabalin after or before the reduction of compound (V).

According to the process of the invention, compound (V) is prepared by hydrolysis of a compound of general formula (IV). In a particular embodiment of the invention the hydrolysis is carried out in a basic medium. According to a preferred embodiment the base is selected from LiOH, NaOH, KOH, Ba(OH)₂ and their mixtures.

According to the process of the invention, compound (IV) is obtained from compound of general formula (III) according to one of the following alternatives:

### Alternative 1):

Treating compound of formula (III) with the mixture resulting from reaction of:a base, preferably with BuLi or *tert*-BuOK; with a compound of formula Ph₃PCH(CH₃)₂X wherein X is Br or I;

### Alternative 2):

Treating compound of formula (III) with the mixture resulting from reaction of:a base, such as BuLi, tert-BuOK or NaH; with(EtO)₂P(O)CH(CH₃)₂, or

### Alternative 3):

a) treating compound of formula (III) with one of the following reagents: i-PrLi or i-PrMgX, wherein X= Cl, Br, I; and
b) subsequent dehydration by HCl, H₂SO₄, *p*-TsOH, or by treatment with mesyl chloride in the presence of pyridine or triethylamine.

According to a particular embodiment of the present invention compound (III) is treated with triphenylphosphonium iodide and BuLi under N₂ atmosphere in the presence of anhydrous THF as solvent.

In a preferred embodiment of the invention compound of formula (III) is N-protected, and thus more soluble under the above mentioned reaction conditions. The resulting product, compound of formula (IV) N-protected, may be easily purified. This constitutes an additional advantage of this embodiment of the process of the invention.

According to the process of the invention, compound (III) is obtained by oxidation of compound of general formula (II). Oxidation is preferably carried out with sodium periodate in a solvent such as MeOH-H₂O.

Compound (II) is obtained by acid hydrolysis of the dioxolan ring of the compound of general formula (I): 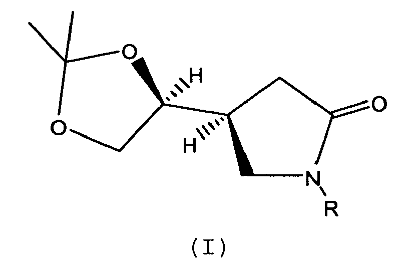 in the presence, for instance, of one of the following reactants: HCl, AcOH, CF₃CO₂H, a sulphonic acid (*p*-TsOH), a sulphonic ion exchange resin, HClO₄, oxalic acid, citric acid or their mixtures in an adequate solvent. In a particular embodiment of the invention, compound (I) is treated with HCl in MeOH. In another particular embodiment of the invention compound (I) is dissolved in methanol and refluxed in the presence of lewatit^{®} (wet) ion-exchange resine.

In another aspect the invention relates to the new intermediates involved in the process of the invention. The intermediates are novel chiral compounds, which may be purified and isolated.

One of the advantages of the process of the invention resides in that the optical purity is maintained through the process.

In this sense the invention relates to a compound of general formula (I): 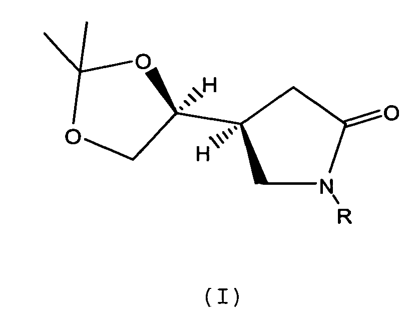 wherein R is a protective group selected from Bn, Boc, Cbz, 4-methoxybenzyl (PMB), and benzyloxymethyl (BOM) is an additional object of the invention.

The invention also relates in another aspect to a compound of general formula (II): 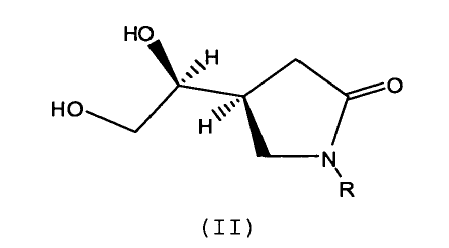 where R represents H, Boc, Cbz, PMB (4-methoxybenzyl), BOM (benzyloxymethyl) is another object of the present invention.

Further, the invention relates to a compound of general formula (III): 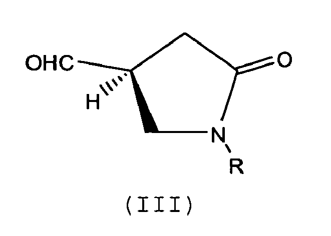 where R represents H, benzyl, Boc, Cbz, PMB or BOM is another object of the present invention.

In an additional aspect the invention relates to a compound of general formula (IV): 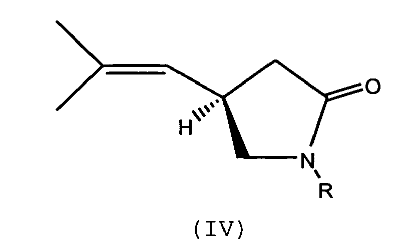 where R represents H, benzyl, Boc, Cbz, PMB, or BOM.

In a further additional aspect the invention relates to a compound of general formula (V): 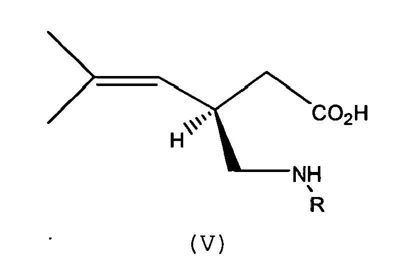 where R represents H, benzyl, Boc, Cbz, PMB, BOM.

The invention relates to the use of a compound of formula (I), 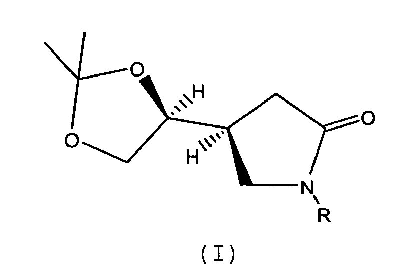 where R represents H, benzyl, Boc, Cbz, PMB, or BOM in the preparation of (S)-pregabalin.

The starting compound may be obtained from D-mannitol bisacetonide, a commercially available product, as shown in the following **Schema 2:**

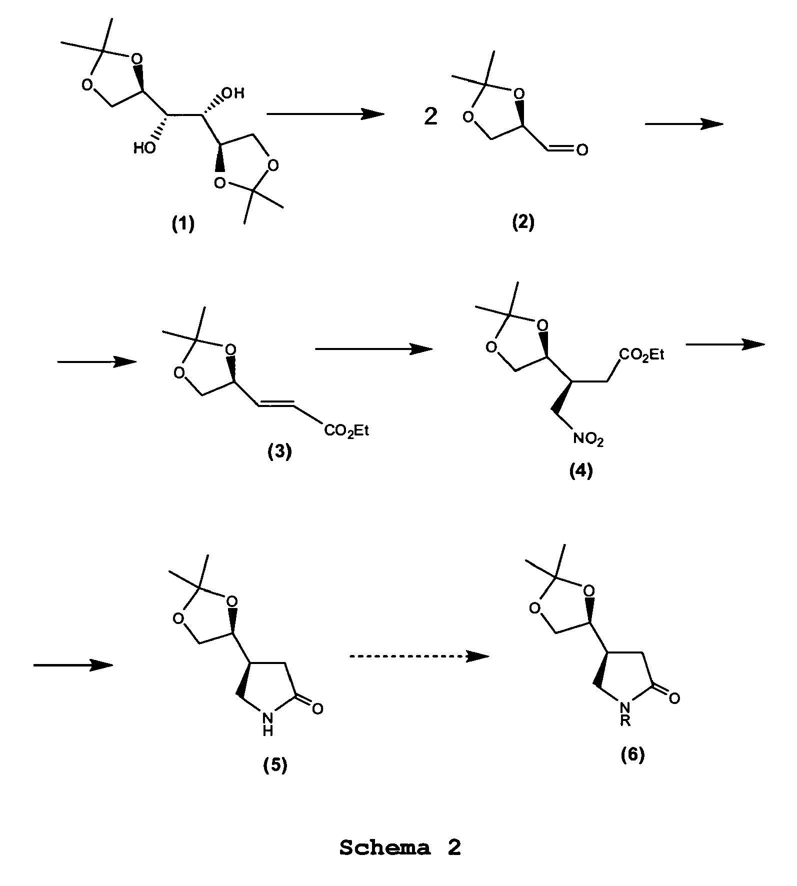

According to this route a compound of formula (I),(compounds (**5**) and (**6**) from **Schema 2)** 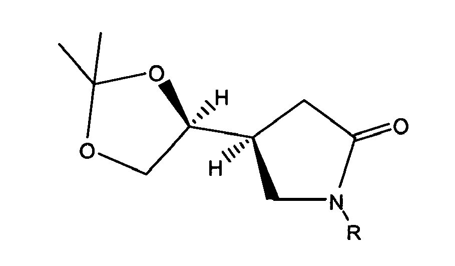 is prepared starting from the precursor D-mannitol bisacetonide (**1**) by oxidation to produce compound (**2**) introducing thus, chirality. According to the stoichiometry of the reaction 1 mol of (**1**) yields 2 moles of (**2**). In a preferred embodiment of the invention, oxidation of D-mannitol bisacetonide is carried out with sodium periodate in an adequate solvent, such as THF-H₂O, preferably in a ratio 9:1.

The compound (**2**) is treated thereafter with triethyl phosphonoacetate ((EtO)₂P(O)CHCO₂Et) and a base such as *t*-BuOK in an inert solvent such as anhydrous dichloromethane and under N₂ atmosphere, to obtain compound (**3**);

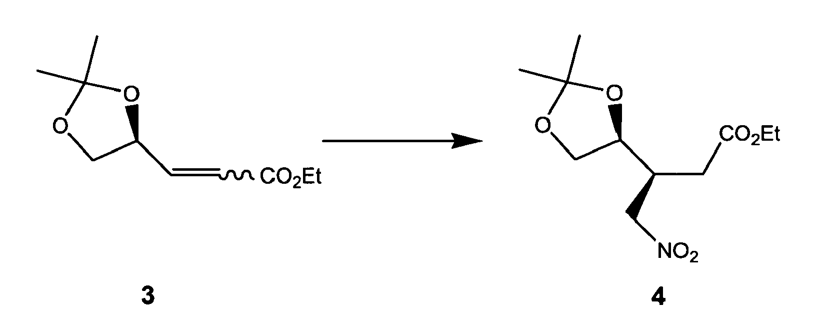

Compound (**3**) is then treated with NO₂CH₃ and tetra-n-butylammonium fluoride (TBAF) in an inert solvent, such as THF, to obtain compound (**4**);

Reduction of compound (**4**) followed by subsequent cyclation yields compound of formula (I), wherein R is H. Compound (**4**) may be purified to obtain a white solid crystal.

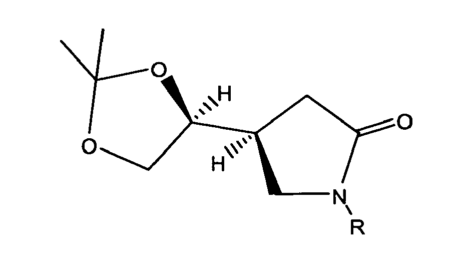

In a particular embodiment of the invention reduction and cyclation are carried out with NH₄HCO₂, over Pd(OH)₂/C as catalyst in the presence of a solvent such as methanol.

Optionally, the free amine group may be further protected with a conventional amine protective group according to known methods to yield a compound of formula (I) wherein R is benzyl, Boc, Cbz, PMB or BOM. The introduction of said protective group presents the additional advantage that some of the intermediates involved in the process of the invention are easier to purify as the corresponding free amine form.

The process of the present invention starts thus with a chiral compound of the above formula, optionally with an amine protective group.

The process of the invention presents several advantages over previous methods of preparing pregabalin. For example there is no need to remove undesired and costly R- enantiomer. It does not require too costly chiral auxiliaries or catalysts and unlike the classical resolution it does not require stoichiometric and costly amounts of a chiral resolution agent.

Another important advantage is the lower cost and easy availability of the starting material D-mannitol bisacetonide. The enantiomeric selection occurs at the beginning of the process with chiral compound (I) and chiralty is retained through the process.
Thus, the present process presents both economical and environmental advantages.

The foregoing is illustrative of the present invention. This invention however is not limited to the following precise embodiments described herein, but encompasses all equivalent modifications within the scope of the claims which follow.

### EXAMPLES

### Example 1: Synthesis of compound (I), wherein R=H 1.1 Synthesis of (R)-2,2-Dimethyl-1,3-dioxolane-4-carbaldehyde (2)

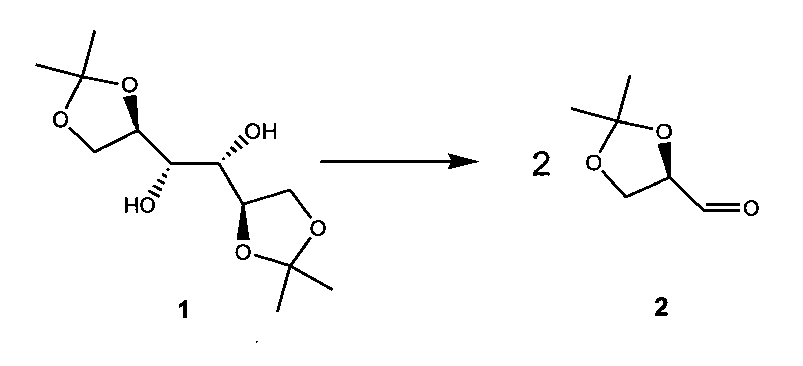

NaIO₄ (16 g, 74.8 mmol) was added to a stirred solution of (**1**) (D-mannitol bisacetonide) (20 g, 76.2 mmol) in 9:1 THF- H₂O (280 mL), and the resultant mixture was stirred for 4 h. The produced precipitate was filtered off, and most of THF was evaporated under reduced pressure. Then water (20 mL) was added and the aqueous solution was extracted with dichloromethane (6 X 50 mL). The combined organic extracts were dried over MgSO₄ and solvent was removed to afford **(2)** ((R)-2,2-Dimethyl-1,3-dioxolane-4-carbaldehyde)(16.8 g, 85% yield), which was used in the next step without further purification.
Characterization of (2):
- **¹H-NMR (250 MHz, CDCl₃)**
   1.4 (s, 3H), 1.5 (s, 3H), 4.1 (m, 2H), 4.4 (m, 1H), 9.7 (d, ³*J_{H-H}* = 2 Hz, 1H).
- **IR (film):** 3417, 2985, 1735, 1372, 1064 cm⁻¹.
- **appearence:** colourless oil.

### 1.2 Synthesis of (E)-3-((S)-2,2-Dimethyl-1,3-dioxolan-4-yl)acrylate (3)

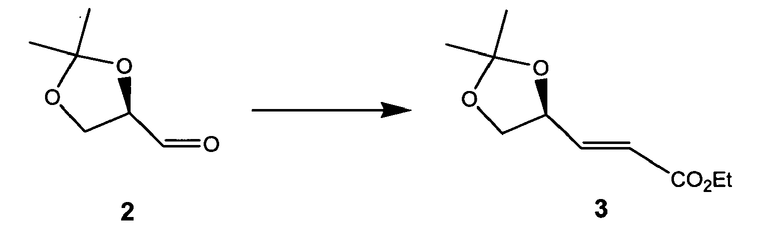

Potassium tert-butoxide (7.8 g, 69.2 mmol) was added to a stirred ice-cooled solution of triethyl phosphonoacetate (13.9 mL, 69.4 mmol) in anhydrous dichloromethane (200 mL) under nitrogen atmosphere. The mixture was stirred at 0°C during 20 min. Then aldehyde **(2)** (7.5 g, 57.7 mmol) in dichloromethane (10 mL) was added and the mixture was stirred at 0°C for 1.5 h. The solvent was evaporated under vacuo and the residue was poured into EtOAc (75 mL) and successively washed with saturated aqueous NaHCO₃. The organic phase was dried over MgSO₄ and the solvent was removed. The residue (10.5 g) was chromatographed on Baker® silica gel using dichloromethane as eluent to give pure **(*E*)-3-((S)-2,2-Dimethyl-1,3-dioxolan-4-yl)acrylate (3)** (8.2 g, 71% yield) as the only or major isomer.
Characterization of (3):
- **¹H-NMR (250 MHz, CDCl₃)**
   1.3 (t, ³*J_{H-H} =* 7 Hz, 3H), 1.4 (s, 3H), 1.5 (s, 3H), 3.7 (dd, ³*J_{H-H}* = 7 Hz, ²*J_{H-H} =* 8.25 Hz, 1H), 4. 2 (m, 3H), 4.7 (m, 2H), 6.1 (dd, ⁴*J_{H-H}* 1.5 Hz, ³*J_{H-H}* = 15.5 Hz, 1H), 6.9 (dd, ³*J_{H-H} =* 5.75 Hz, *³J_{H-H} =* 15.5 Hz, 1H).
- **¹³C-NMR (62.5MHz, CDCl₃)**
   14.1 (1C), 25.6 (1C), 26.3 (1C), 60.4 (1C), 68.7 (1C), 74.8 (1C), 110.0 (1C), 122.3 (1C), 144.5 (1C), 165.8 (1C).
- **IR (film):** 2985, 1772, 1662, 1371, 1059 cm⁻¹.
- **appearence:** colourless oil.

### 1.3 Synthesis of (S)-Ethyl 3-((S)-2,2-dimethyl-1,3-dioxolan-4-yl)-4-nitrobutanoate (4)

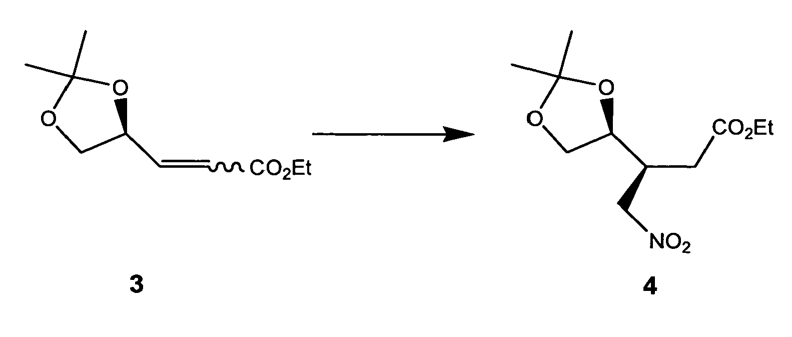

Nitromethane (3.5 mL, 64.6 mmol) and TBAF (tetrabutyl ammonium fluoride) 1 M solution in THF (64.6 mL, 64.6 mmol) were successively added to a stirred cooled (-5°C) solution of a Z/E mixture of alkenoates **(3)** (9.5 g, 47.4 mmol) in THF (250 mL). The mixture was stirred at -5°C for 3 h. The solvent was evaporated under vacuo and the residue was poured into EtOAc (150 mL) and successively washed with saturated aqueous NaHCO₃ and brine. The organic phase was dried over MgSO₄ and the solvent was removed. The residue (12.5 g) was chromatographed on Baker® silica gel using dichloromethane as eluent to afford pure **(4) (*S*)-Ethyl 3-((S)-2,2-dimethyl-1,3-dioxolan-4-yl)-4-nitrobutanoate** (10.3 g, 83% yield).
Characterization of (4):
- **¹H-NMR (250 MHz, CDCl₃)**
   1.1 (t, ³*J_{H-H} =* 7 Hz, 3H), 1.3 (s, 3H), 1.4 (s, 3H), 2.5 (m, 2H), 2.7 (m, 1H), 3.7 (m, 1H), 4.1 (m, 4H), 4.6 (m, 2H).
- **¹³C-NMR (62.5MHz, CDCl₃)**
   14.1 (1C), 24.9 (1C), 26.3 (1C), 33.5 (1C), 37.8 (1C), 60.9 (1C), 67.3 (1C), 75.3 (1C), 75.4 (1C), 109.6 (1C), 170.8 (1C).
- **IR (film):** 2985, 1730, 1552, 1373, 852 cm⁻¹.
- **appearence:** colourless oil.

### 1.4 Synthesis of (S)-4-((S)-2,2-Dimethyl-1,3-dioxolan-4-yl)pyrrolidin-2-one (I)

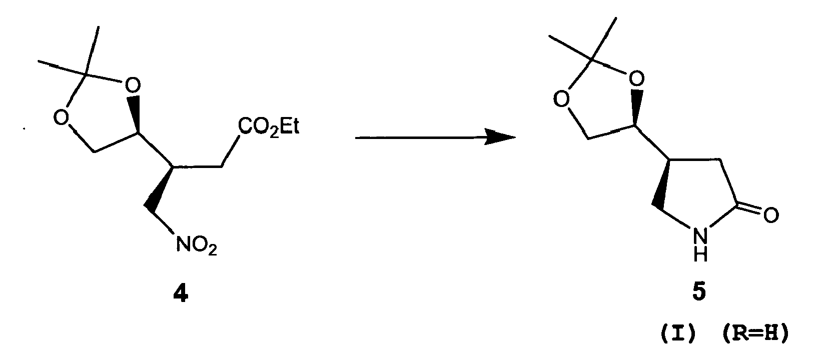

Ammonium formate (4.2 g, 66.6 mmol) and 20% Pd(OH)₂/C (2.3 g) were successively and carefully added to a solution of **(4)** (3.8 g, 14.6 mmol) in methanol (200 mL). The mixture was heated to reflux overnight. The catalyst was removed by filtration through Celite®, washed with methanol and chromatographed on Baker® silica gel using EtOAc as eluent to afford pure **(I) (*S*)-4-((*S*)-2,2-Dimethyl-1,3-dioxolan-4-yl)pyrrolidin-2-one** (2.3 g, 85% yield).
Characterization of (I) (R=H):
- **¹H-NMR (250 MHz, CDCl₃)**
   1.4 (s, 3H), 1.5 (s, 3H), 2.1 (dd, ³*J_{H-H}* = 9.25 Hz, ²*_{H-H}* = 17.25 Hz, 1H), 2.4 (dd, ³*J_{H-H} =* 9 Hz, ²*J_{H-H}* = 17 Hz, 1H)), 2.6 (m, 1H), 3.4 (dd, *³J_{H-H} =* 5.75 Hz, ²*J_{H-H} =* 9.75 Hz, 1H), 3.6 (m, 2H), 4.1 (m, 2H), 6.0 (broad singlet, 1H).
- **¹³C-NMR (62.5 MHz, CDCl₃)**
   25.7 (1C), 27.0 (1C), 33.0 (1C), 38.1 (1C), 44.9 (1C), 67.9 (1C), 77.7 (1C), 109.5 (1C), 177.5 (1C).
- **IR (solid):** 3229, 2985, 1687, 1371, 1051 cm⁻¹.
- **mp=** 84-86°C (EtOAc/pentane)
- **appearance:** white solid, crystals.

### Example 2

### Synthesis of (S)-pregabalin [intermediate (IV)] (Schema 1)

### 2.1 Synthesis of (S)-4-((S)-1,2-Dihydroxyethyl)pyrrolidin-2-one (II)

A solution of **(*S*)-4-((S)-2,2-Dimethyl-1,3-dioxolan-4-yl)pyrrolidin-2-one** (I) (1.5 g, 8.1 mmol) in methanol (25 mL) was refluxed for 4 h in the presence of lewatit® (wet) ion-exchange resin (30 mg). The reaction mixture was filtered off, and the filtrate was concentrated under vacuo to afford **(*S*)-4-((*S*)-1,2-Dihydroxyethyl)pyrrolidin-2-one** (II) (1.1 g, 100% yield).
Characterization of (II) (R=H):
- **¹H-NMR (250 MHz, MeOD-d₄)**
   2.4 (m, 2H), 2.7 (m, 1H), 3.6 (m, 5H).
- **¹³C-NMR (62.5 MHz, MeOD-d₄**)
   35.3 (1C), 39.3 (1C), 46.4 (1C), 66.6 (1C), 75.7 (1C), 181.3 (1C).
- **IR (film):** 3354, 3248, 2914, 1650, 1021 cm⁻¹.
- **mp=** 84-86°C (EtOAc/pentane)
- **appearance:** white solid.

### 2.2 Synthesis of (S)-5-Oxopyrrolidine-3-carbaldehyde (III)

NaIO₄ (1.2 g, 5.6 mmol) was added to a stirred solution of **(*S*)-4-((*S*)-1,2-Dihydroxyethyl)pyrrolidin-2-one (II)** (680 mg, 4.7 mmol) in 4:1 Methanol - H₂O (45 mL), and the resultant mixture was stirred at 0°C for 30 min. The produced precipitate was filtered off, and solvent was removed to afford **(*S*)-5-Oxopyrrolidine-3-carbaldehyde (III)** (529 mg, 100% yield), which was used in the next step without further purification.
Characterization of (III) (R=H):
- **¹H-NMR (250 MHz, acetone-d₆)**
   2.5 (m, 2H), 3.2 (m, 1H), 3.6 (m, 2H), 6.7 (broad singlet, 1H), 9.7 (d, ³*J_{H-H} =* 1 Hz, 1H).
- **appearance:** light yellow oil.

### 2.3 Synthesis of (R)-4-(2-Methylprop-1-enyl)pyrrolidin-2-one (IV)

n-BuLi 2.5 M in hexanes (2.5 mL, 6.2 mmol) was added to a stirred ice-cooled solution of triphenylphosphonium iodide (1.9 g, 4.5 mmol) in anhydrous THF (60 mL) under nitrogen atmosphere. The mixture was stirred at 0°C for 1 h. Then a solution of **(*S*)-5-Oxopyrrolidine-3-carbaldehyde (III)** (200 mg, 1.8 mmol) in THF (10+5 mL) was added *via canula,* and the mixture was allowed to arrive to room temperature and stirred for 3 h. The solvent was evaporated under pressure and the residue was poured into EtOAc (25 mL) and was successively washed with saturated aqueous NaHCO₃. The organic phase was dried over MgSO₄ and solvent was removed. The residue (320 mg) was chromatographed on Baker® silica gel using EtOAc as eluent to afford pure **(*R*)-4-(2-Methylprop-1-enyl)pyrrolidin-2-one (IV)** (100 mg, 41% yield).
Characterization of (IV) (R=H):
- **¹H-NMR (250 MHz, CDCl₃)**
   1.7 (d, *⁴J_{H-H}* = 1.25 Hz, 3H), 1.8 (d, *⁴J_{H-H}* = 1. 25 Hz, 3H), 2.1 (dd, *³J_{H-H}* =8.75 Hz, *²J_{H-H}* = 17 Hz, 2H), 2.5 (dd, *³J_{H-H}* =8.75 Hz, *²J_{H-H}* =16.5 Hz, 1H), 3.0 (dd, *³J_{H-H}* = 7 Hz, *²J_{H-H}* = 9 Hz, 1H), 3.3 (sept, *J_{H-H} =* 9 Hz, 1H), 3.5 (t, *J_{H-H} =* 7.5 Hz, 1H), 5.1 (d, *³J_{H-H}* = 7.5 Hz, 1H), 6.1 (broad singlet, 1H).
- **¹³C-NMR (62.5 MHz, CDCl₃)**
   18.0 (1C), 25.5 (1C), 34.2 (1C), 37.2 (1C), 48.3 (1C), 125.5 (1C), 131.4 (1C), 178.3 (1C).
- **appearance:** colourless oil.

### Example 3

### Synthesis of (S)-1-Benzyl-4-((S)-2,2-dimethyl-1,3-dioxolan-4-yl)pyrrolidin-2-one of formula (I), wherein R is Bn

Sodium hydride in paraffin oil (60%, 0.24 g, 6.0 mmol) was added to a stirred solution of **(*S*)-4-((*S*)-2,2-Dimethyl-1,3-dioxolan-4-yl)pyrrolidin-2-one (I)** (1.0 g, 5.4 mmol) in anhydrous THF (75 mL) over an ice bath. The mixture was stirred for 30 min at 0°C and benzyl bromide (0.7 mL, 5.7 mmol) was added dropwise. The reaction mixture was allowed to warm to room temperature and was stirred for a further 3 h. The solvent was evaporated under vacuo and the residue poured into EtOAc (100 mL) and washed several times with water. The organic phase was dried over MgSO₄ and the solvent was removed. The residue (0.8 g) was chromatographed on Baker® silica gel EtOAc-hexane (2:3) as eluent to afford pure **(*S*)-1-Benzyl-4-((*S*)-2,2-dimethyl-1,3-dioxolan-4-yl)pyrrolidin-2-one [compound of formula (I), wherein R is benzyl]** (0.5 g, 34% yield).
Characterization of (I) (R=Bn):
- **¹H-NMR (250 MHz, CDCl₃)**
   1.3 (s, 3H), 1.4 (s, 3H), 2.2 (m, 1H), 2.5 (m, 2H), 3.3 (m, 2H), 3.5 (m, 1H), 4.0 (m, 2H), 4.3 (d, *²J_{H-H}* = 12 Hz), 4.5 (d, *²J_{H-H}* = 12 Hz), 7. 3 (m, 5H).
- **¹³C-NMR (62.5 MHz, CDCl₃)**
   25.0 (1C), 26.3 (1C), 33.0 (1C), 34.0 (1C), 46.1 (1C), 48.3 (1C), 67.0 (1C), 77.1 (1C), 109.0 (1C), 127.3 (1C), 127.8 (2C), 128.4 (2C), 136.0 (1C), 173.3 (1C).
- **IR (film):,** 2983, 2240, 1682, 1427, 1062 cm⁻¹.
- **appearence:** colourless oil.

### Example 4

### Synthesis of (S)-pregabalin [intermediate (IV)] (Schema 1)

### 4.1 Synthesis of (S)-1-Benzyl-4-((S)-1,2-dihydroxyethyl)pyrrolidin-2-one (II)

A solution of **(*S*)-1-Benzyl-4-((*S*)-2,2-dimethyl-1,3-dioxolan-4-yl)pyrrolidin-2-one [(I), wherein the protective group R is benzyl]** (600 mg, 2.2 mmol) in methanol (25 mL) was refluxed for 4 h in the presence of lewatit® (wet) ion-exchange resin (30 mg). The reaction mixture was filtered off, and the filtrate was concentrated under vacuo to afford **(*S*)-1-Benzyl-4-((*S*)-1,2-dihydroxyethyl)pyrrolidin-2-one (II)** (510 mg, 100% yield).
Characterization of (II) (R=Bn):
- **¹H-NMR (250 MHz, MeOD-d₄)**
   1.9 (broad singlet, 2H), 2.3 (m, 1H), 2.5 (m, 2H), 3.4 (m, 3H), 3.7 (m, 2H), 4.5 (m, 2H), 7.3 (m, 5H).
- **appearence:** light yellow oil.

### 4.2 Synthesis of (S)-1-Benzyl-5-oxopyrrolidine-3-carbaldehyde (III)

NaIO₄ (375 mg, 1.8 mmol) was added to a stirred solution of **(*S*)-1-Benzyl-4-((*S*)-1,2-dihydroxyethyl)pyrrolidin-2-one (II)** (340 mg, 1.5 mmol) in 4:1 methanol - H₂O (10 mL), and the resultant mixture was stirred at 0°C for 30 min. The produced precipitate was filtered off, and most of methanol was evaporated under reduced pressure. Then 15 mL of water was added and the water solution was extracted with EtOAc (4 X 20 mL). The combined organic extracts were dried over MgSO₄ and solvent was removed to afford **(*S*)-1-Benzyl-5-oxopyrrolidine-3-carbaldehyde (III)** (290 mg, 100% yield), which was used in the next step without further purification.
Characterization of (III) (R=Bn):
- **¹H-NMR (250 MHz, acetone-d₆)**
   2.6 (m, 1H), 3.3 (m, 2H), 3.6 (m, 2H), 4.5 (s, 2H), 7.3 (m, 5H), 9.7 (d, ³*J_{H-H} =* 1.5 Hz, 1H).
- **appearence:** yellow oil.

### 4.3 Synthesis of (R)-1-Benzyl-4-(2-methylprop-1-enyl)pyrrolidin-2-one (IV)

n-BuLi 2.5 M in hexanes (2.9 mL, 7.3 mmol) was added to a stirred ice-cooled solution of triphenylphosphonium iodide (3.1 g, 7.1 mmol) in anhydrous THF (20 mL) under nitrogen atmosphere. The mixture was stirred at 0°C for 1 h. Then a solution of **(*S*)-1-Benzyl-5-oxopyrrolidine-3-carbaldehyde (III)** (290 mg, 1.4 mmol) in THF (10+5 ml) was added *via canula,* and the mixture was allowed to arrive to room temperature and stirred for 3 h. The solvent was evaporated under pressure and the residue was poured into EtOAc (25 mL) and was successively washed with saturated aqueous NaHCO₃. The organic phase was dried over MgSO₄ and solvent was removed. The residue (430 mg) was chromatographed on Baker® silica gel using EtOAc-hexane (2:3) as eluent to afford pure **(*R*)-1-Benzyl-4-(2-methylprop-1-enyl)pyrrolidin-2-one (IV)** (196 mg, 60% yield).
Characterization of (IV) (R=Bn):
- **¹H-NMR (250 MHz, CDCl₃)**
   1.6 (d, ³*J_{H-H} =* 1Hz, 3H), 1.7 (d, ³*J_{H-H}*= 1Hz, 3H), 2.2 (dd, *³J_{H-H} =* 7.75 Hz, ²*J_{H-H}* = 15.5 Hz, 1H), 2.6 (dd, *³J_{H-H}=* 7.75 Hz, ²*J_{H-H}* = 16.25 Hz, 1H), 2.9 (dd, ³*J_{H-H} =* 6.75 Hz, ²*J_{H-H} =* 9.25 Hz, 1H), 3.2 (m, 1H), 3.4 (dd, ³*J_{H-H} =* 8.0 Hz, ²*J_{H-H}* = 9.5 Hz, 1H), 4.4 (d, ²*J_{H-H}=* 21.25 Hz, 1H), 4.5 (d, ²*J_{H-H}=* 21.5 Hz, 1H), 5.1 (c.a., 1H), 7.3 (m, 5H).
- **¹³C-NMR (62.5 MHz, CDCl₃)**
   17.9 (1C), 25.4 (1C), 30.9 (1C), 38.2 (1C), 46.3 (1C), 52.5 (1C), 125.7 (1C), 127.4 (1C), 128.0 (2C), 128.7 (2C), 133.8 (1C), 136.4 (1C), 174.2 (1C).
- **appearence:** colourless oil.

### Example 5

### Synthesis of (S)-tert-Butyl 4-((S)-2,2-dimethyl-1,3-dioxolan-4-yl)-2-oxopyrrolidine-1-carboxylate of formula (I) wherein R is Boc

To a stirred solution of **(*S*)-4-((*S*)-2,2-dimethyl-1,3-dioxolan-4-yl)pyrrolidin-2-one (I)** (500 mg, 2.7 mmol) in dichloromethane (25 mL) were successively added triethylamine (0.4 mL, 2.9 mmol), 4-dimethylaminopyridine (330 mg, 2.7 mmol) and Boc anhydride (1.2 mL, 5.4 mmol). The light-protected mixture was stirred at room temperature for 12 h. The solvent was evaporated under vacuo and the residue was chromatographed on Baker® silica gel using EtOAc-hexane (1:2) as eluent to afford pure **(*S*)-*tert*-butyl 4-((*S*)-2,2-dimethyl-1,3-dioxolan-4-yl)-2-oxopyrrolidine-1-carboxylate [compound of formula (I), wherein R is Boc]** (770 mg, 100% yield), that can be crystallized in EtOAc/pentane.
Characterization (I) (R=Boc)
- **¹H-NMR (250 MHz, CDCl₃)**
   1.4 (s, 3H), 1.5 (s, 3H), 1.6 (s, 9H), 2.3 (m, 1H), 2.4 (m, 1H), 2.6 (m, 1H), 3.7 (m, 2H), 3.9 (dd, *³J_{H-H}* = 6 Hz, *³J_{H-H} =* 10.5 Hz, 1H), 4.1 (m, 2H).
- **¹³C-NMR (62.5 MHz, CDCl₃)**
   25.2 (1C), 26.6 (1C), 28.0 (1C), 33.8 (1C), 35.5 (1C), 48.4 (1C), 67.5 (1C), 77.1 (1C), 82.9 (1C), 109.7 (1C), 149.9 (1C), 172.7 (1C).
- **IR (solid):** 2982, 1789, 1686, 1149, 1044 cm⁻¹.
- **mp=** 106-108°C (EtOAC/pentane).
- **appearance:** white solid, crystals.

### Example 6

### Synthesis of (S)-pregabalin hydrochloride

### 6.1 Synthesis of intermediate (R) -tert-Butyl 4- (2-methylprop-1-enyl)-2-oxopyrrolidine-1-carboxylate [compound (IV), wherein R=Boc]

(R)-tert-Butyl 4-(2-methylprop-1-enyl)-2-oxopyrrolidine-1-carboxylate was prepared from compound of formula (I) (R=Boc) which was prepared according to Example 5, and following the method as described in Example 2 (2.2 and 2.3).
Characterization of (IV) (R=Boc)
- **¹H-NMR (250 MHz, CDCl₃)**
   1.5 (s, 9H), 1.6 (d, *⁴J_{H-H}* = 1 Hz, 3H), 1.7 (d, *⁴J_{H-H}* = 1 Hz, 3H), 2.3 (dd, *³J_{H-H}* = 9.5 Hz, ²*J_{H-H}* = 17 Hz, 1H), 2.6 (dd, *³J_{H-H}* = 8 Hz, *²J_{H-H}* = 17 Hz, 1H), 3.2 (sextuplet, *³J_{H-H}* = 8.5 Hz, 1H), 3.3 (dd, *³J_{H-H}* = 8 Hz, *²J_{H-H}* = 10.5 Hz, 1H), 3.9 (dd, *³J_{H-H}* = 8 Hz, *²J_{H-H}* = 10.5 Hz, 1H), 5.1 (c.a., 1H).
- **¹³C-NMR (62.5 MHz, CDCl₃)**
   18.2 (1C), 25.7 (1C), 28.2 (3C), 30.5 (1C), 40.2 (1C), 52.2 (1C), 82.8 (1C), 124.4 (1C), 135.3 (1C), 150.1 (1C), 173.6 (1C).
- **IR (solid):** 2978, 2931, 1786-1751, 1713, 1455, 1084 cm⁻¹.

### 6.2 Synthesis of (R)-3-((tert-Butoxycarbonylamino)methyl)-5-methylhex-4-enoic acid [compound (V), wherein R=Boc]

To a 0.2 M solution of **(R)-tert-butyl 4-(2-methylprop-1-enyl)-2-oxopyrrolidine-1-carboxylate [compound (IV), wherein R=Boc]** (100 mg, 0.4 mmol) in tetrahydrofuran was added 1.3 mL ( 1.3 mmol) of a 1.0 N solution of lithium hydroxide. The solution was stirred for 4 hours at room temperature. After removal of tetrahydrofuran in vacuo, the basic aqueous phase was acidified by the addition of 10% acetic acid and extracted with ethyl acetate. The combined organic extracts were dried over MgSO₄ and solvent was removed to afford **(R)-3-((tert-butoxycarbonylamino)methyl)-5-methylhex-4-enoic acid [compound (V), wherein R=Boc]** (106 mg, 100% yield), which was used in the next step without further purification.
Characterization of (V) (R=Boc)
- **¹H-NMR (250 MHz, CDCl₃)**
   1.5 (s, 9H), 1.7 (s, 3H), 1.8 (s, 3H), 2.3 (m, 2H), 3.0 (m, 2H), 3.2 (m, 1H), 4.7 (b.s, 1H), 4.9 (c.a., 1H).
- **¹³C-NMR (62.5 MHz, CDCl₃)**
   18.2 (1C), 25.9 (1C), 28.4 (3C), 29.7 (1C), 37.9 (1C), 44.5 (1C), 79.4 (1C), 124.2 (1C), 135.5 (1C), 156.2 (1C), 177.1 (1C).
- **IR (film): 3326,** 2969, 2855, 1707, 1644, 1166, 1081 cm⁻¹.
- **HRMS** : **ESI-QTOF (MeOH)**
   Mass Calcd for **C₁₃H₂₃NO₄ + Na:** 280.1519 g/mol Found for **C₁₃H₂₃NO₄ + Na:** 280.1526 g/mol
- **appearance:** light yellow dense oil.

### 6.3 Synthesis of (S)-3-(Aminomethyl)-5-methylhexanoic acid hydrochloride, pregabalin hydrochloride

6N HCl (0.3 mL, 1.8 mmol) was added to a solution of **(*R*)-3-((*tert*-butoxycarbonylamino)methyl)-5-methylhex-4-enoic acid [compound (V), wherein R=Boc]** (106 mg, 0.4 mmol) in methanol (5 mL) and the mixture was hydrogenated over 20% Pd(OH)₂ /C (40 mg) at room temperature and at 6 atm for 12 h. The catalyst was removed by filtration and washed with methanol. The filtrate was evaporated to afford pure **(S)-3-(aminomethyl)-5-methylhexanoic acid hydrochloride** (82 mg, 100% yield).
Characterization of (S)-pregabalin hydrochloride:
- **¹H-NMR (250 MHz, D₂O)**
   0.8 (t, *³J_{H-H}* = 6 Hz, 6H), 1.2 (t, *³J_{H-H}* = 7.5 Hz, 2H), 1. 6 (sept, *³J_{H-H}* = 6 Hz, 1H), 2.2 (m, 1H), 2.4 (t, *³J_{H-H}* = 6 Hz, 2H), 2.9 (d, *³J_{H-H} =* 6. 5 Hz, 2H).
- **appearance:** white solid.

## Claims

1. Process for enantioselectively preparing (S)-pregabalin, or a pharmaceutically acceptable acid addition salt, comprising the reduction of a compound of general formula (V) 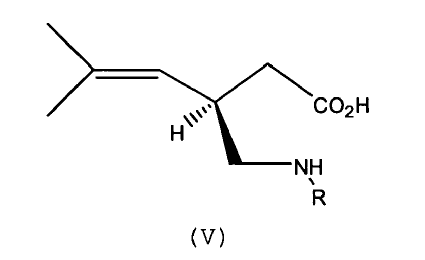 where R represents H or a protective group of the amine.

2. Process for enantioselectively preparing (S)-pregabalin according to claim 1, where the protective group of the amine is benzyl, Boc, Cbz, PMB or BOM.

3. Process for enantioselectively preparing (S)-pregabalin according to claim 1 or 2, where the reduction is carried out:
(i) with hydrogen in the presence of a palladium or platimum catalyst, or
(ii) by hydrogen transfer from HCO₂NH₄ or cyclohexadiene in the presence of a palladium or platinum catalyst, or
(iii) by hydrogenation in the presence of a palladium or platinum catalyst and HCl.

4. Process according to claim 3 wherein the palladium or platinum catalyst is selected from Pd/C, Pd(OH)₂/C, PdCl₂/C, PtO₂/C and their mixtures.

5. Process for enantioselectively preparing (S)-pregabalin according to anyone of claims 1 - 4, where compound (V) is prepared by hydrolysis of a compound of general formula (IV)

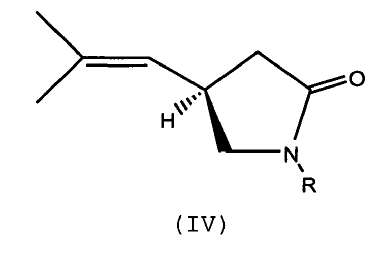

6. Process for enantioselectively preparing (S)-pregabalin according to claim 5, where the hydrolysis is carried out with a base.

7. Process for enantioselectively preparing (S)-pregabalin according to claim 6, where the base is selected from LiOH, NaOH, KOH, Ba(OH)₂ and their mixtures.

8. Process for enantioselectively preparing (S)-pregabalin according to anyone of claims 5-7, where compound (IV) is obtained 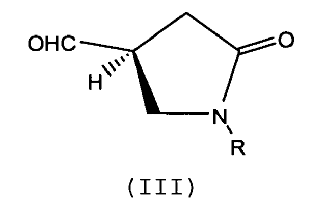 by treating compound of formula (III) with the mixture resulting from reaction of a base with a compound of formula Ph₃PCH(CH₃)₂X wherein X is Br or I.

9. Process according to claim 8, wherein the base is BuLi or *tert*-BuOK.

10. Process for enantioselectively preparing (S)-pregabalin according to anyone of claims 5-7, where compound (IV) is obtained by treating compound of formula (III) with the mixture resulting from reaction of a base with (EtO)₂P(O)CH(CH₃)₂.

11. Process according to claim 10, wherein the base is selected from BuLi, *tert*-BuOK, and NaH.

12. Process for enantioselectively preparing (S)-pregabalin according to anyone of claims 5-7, where compound (IV) is obtained by
a) treating compound of general formula (III) with one of the following reagents i-PrLi or i-PrMgX, wherein X is Cl, Br or I; and
b) subsequent dehydration by HCl,H₂SO₄, *p*-TsOH, or by treatment with mesyl chloride in the presence of pyridine or triethylamine.

13. Process for enantioselectively preparing (S)-pregabalin according to anyone of claims 8-12, where compound (III) is obtained by oxidation of a compound of general formula (II)

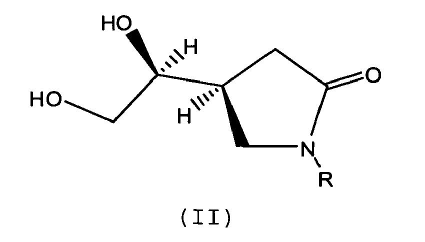

14. Process for enantioselectively preparing (S)-pregabalin according to claim 13, where oxidation is carried out with sodium periodate.

15. Process for enantioselectively preparing (S)-pregabalin according to claim 13 or 14, where compound (II) is obtained by acid hydrolysis of the dioxolan ring of the compound of general formula (I):

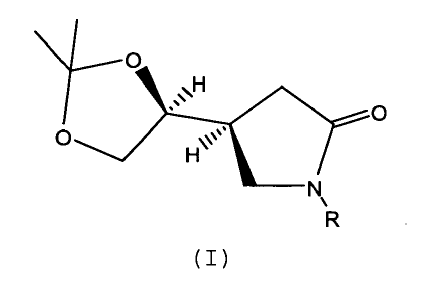

16. Process for enantioselectively preparing (S)-pregabalin according to claim 15, where the acid hydrolysis is carried out in the presence of one of the following reactants: HCl, AcOH, CF₃CO₂H, a sulphonic acid (*p*-TsOH), a sulphonic ion exchange resin, HClO₄, oxalic acid, citric acid or their mixtures.

17. A process for enantioselectively preparing (S)-pregabalin according to anyone of the previous claims, comprising the steps of:
**a)** acid hydrolysis of the dioxolan ring of compound of general formula (I) 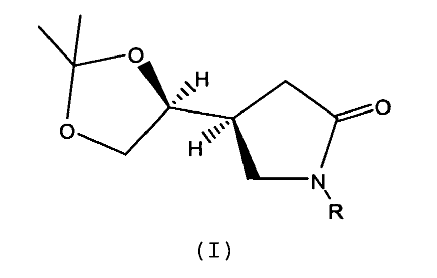 to obtain a compound of general formula (II) 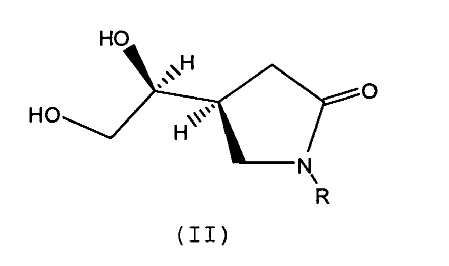
**b)** oxidation of compound (II) to obtain a compound of general formula (III) 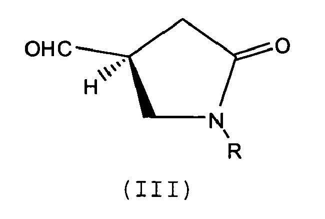
**c)** transforming compound (III) into compound of general formula (IV) 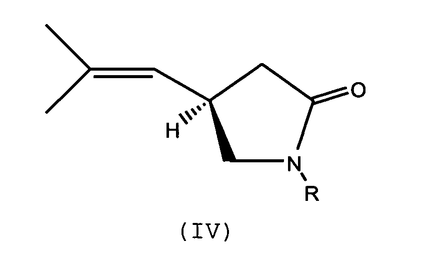 by reacting compound (III) according to any of the following alternatives:
A) with a base and a compound of formula Ph₃PCH(CH₃)₂X, wherein X is Br or I,
B) with a base and (EtO)₂P(O)CH(CH₃)₂; or
C) with one of the following reagents i-PrLi or *i-*PrMgX, wherein X is Cl, Br or I; and
subsequent dehydration with HCl,H₂SO₄, *p*-TsOH, or by treatment with mesyl chloride in the presence of pyridine or triethylamine.
**d)** Subjecting compound (IV) to acid or basic hydrolysis to obtain a compound of general formula (V) 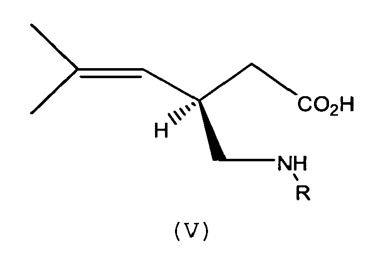
**e)** reducing compound (V) to obtain enantiomerically pure S-pregabalin; and
**f)** optionally converting (S)-pregabalin in a pharmaceutically acceptable acid addition salt thereof.

18. A compound of general formula (I): 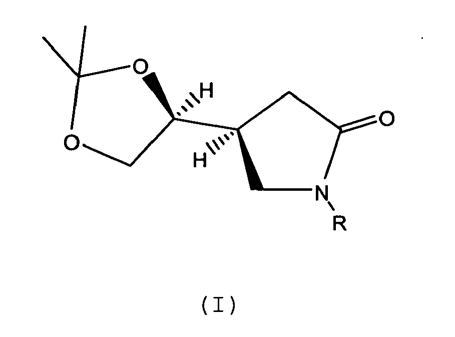 wherein R is a protective group selected from Bn, Boc, Cbz, PMB or BOM.

19. A compound of general formula (II): 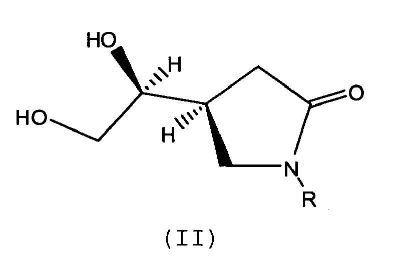 where R represents H, Boc, Cbz, PMB or BOM.

20. A compound of general formula (III): 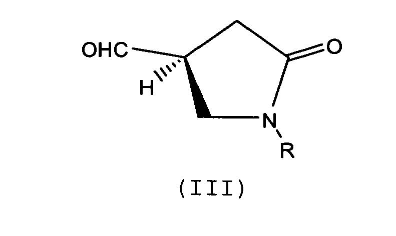 where R represents H, benzyl, Boc, Cbz, PMB or BOM.

21. A compound of general formula (IV): 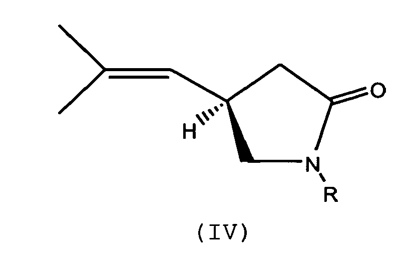 where R represents H, benzyl, Boc, Cbz, PMB or BOM.

22. A compound of general formula (V): 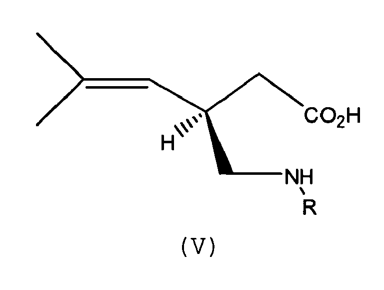 where R represents H, benzyl, Boc, Cbz, PMB or BOM.

23. Use of a compound of formula (I), 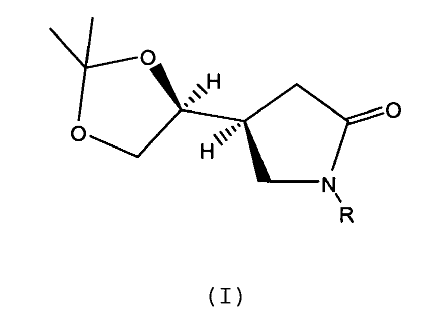 where R represents H, benzyl, Boc, Cbz, PMB or BOM in the preparation of (S)-pregabalin.

24. Process for enantioselectively preparing (S)-pregabalin according to anyone of claims 1-17, where the compound of formula (I), is prepared according to the following steps:
(i) oxidation of D-mannitol bisacetonide (**1**); 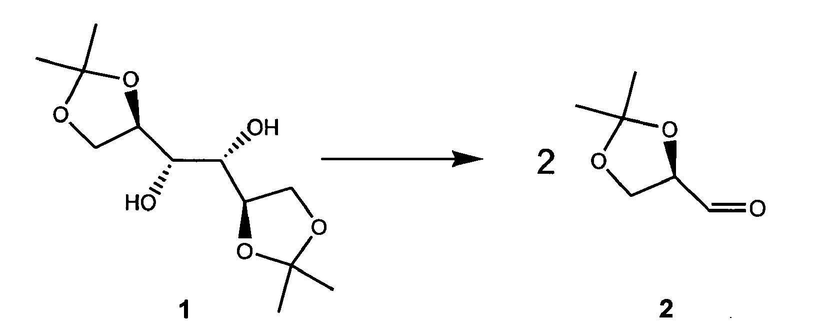
(ii) treatment of compound (**2**) obtained in step (i) with a base and triethyl phosphonoacetate to obtain compound (**3**); 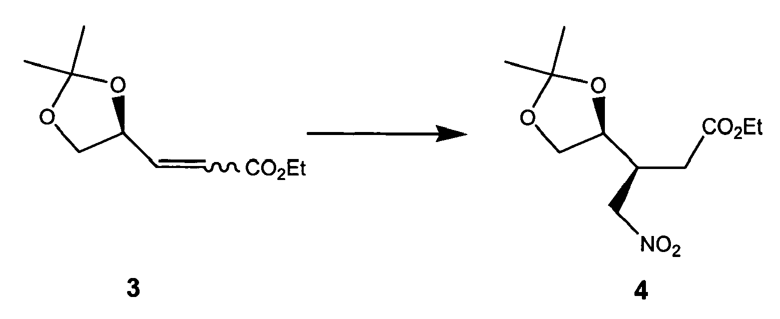
(iii) treating compound (**3**) obtained in step (ii) with NO₂CH₃ and tetra-n-butylammonium fluoride to obtain compound (**4**);
(iv) reduction of compound (**4**) followed by subsequent cyclation to yield compound (I), 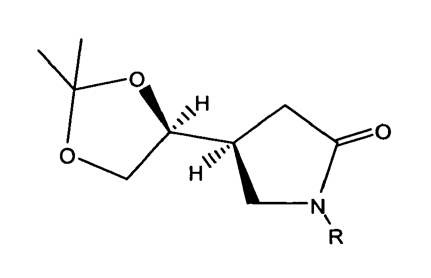
(v) wherein R=H; and
(vi) optionally, protecting the compound obtained in step (v) with a protective group -R.
